# EUROPEAN PATENT APPLICATION

(11) **EP 1 629 710 A1**
(43) Date of publication of application: **01.03.2006**
(21) Application number: 04702789.1
(22) Date of filing: 16.01.2004
(51) Int. Cl.: A01H 1/02

(54) **METHOD OF CROSSING FLOWER COLOR GENOTYPES**

(30) Priority: 22.05.2003 JP 2003144406
(71) Applicant: Hashimoto, Fumio, Kagoshima-shi, Kagoshima 890-0081 (JP); Sakata, Yusuke, Kagoshima-shi, Kagoshima 890-0053 (JP)
(72) Inventor: Hashimoto, Fumio, Kagoshima-shi, Kagoshima 890-0081 (JP); Sakata, Yusuke, Kagoshima-shi, Kagoshima 890-0053 (JP)
(74) Representative: Strehl Schübel-Hopf & Partner
(86) International application number: PCT/JP2004/000297
(87) International publication number: WO 2004/103065

(57) **Abstract**

It is intended to clarify heredity of the biosynthesis of flower pigments and the relationship between flower color heredities and pigment genotypes, thereby providing a method of crossing flower color genotypes which is practically available in creating a novel flower color. There is found out a novel rule that flower color genotypes relate to the biosynthesis of flavonoids represented by the pathway (I) and the heredities of flavonoid 3'-hydroxylase (F3'H) and flavonoid 3',5'-hydroxylase (F3',5'H) are controlled by 5 multiple alleles. As a result, it becomes possible to provide a method of producing a novel flower color with the use of genotypes D/d.E/e.H<X>H<X>.Pg/pg.Cy/cy.Dp/dp by which flower colors can be freely created based on flower pigment genotypes without resort to gene recombination or mutation caused by exposure to radiation, etc.

## Description

### Field of the Invention

The present invention relates to a method for breeding flowering plants with new flower color applied to pigment genotypes of flowering plants. More specifically, the present invention relates to novel plants and process for obtaining novel plants comprising flowers of flowering plants, i.e., flowers of angiosperms, and a crossing method for modifying their genotypes. Also, the present invention concerns a method utilizing plants or parts of plants obtained by breeding, which includes a sexual hybridization stage. Also, the present invention relates to new plants and a method for obtaining the same, i.e., relates to novel flowering plants such as angiosperms, particularly flowers.

### BACKGROUND ARTS

Anthocyanins are one of flavonoids, are broadly existing in flowers, fruits, leaves and the like of plants, and pigment glycosides relating to coloration of red, purple, blue, and the like. When anthocyanins are hydrolyzed with hydrochloric acid, they are decomposed into sugars and anthocyanidin, which is an aglycone (Non-Patent Document 1: Takao Murakami, Constructions and Chemicals of Natural Originated Substance; Hirokawa Shoten, Sept. 1994: 170-172).

Flavonol glycosides are one of flavonoids, is broadly existing in flowers, fruits, leaves and the like of plants, and is a pigment glycoside relating to coloration of yellow. When flavonol is hydrolyzed with hydrochloric acid, it is decomposed into sugars and flavonol, which is an aglycone (Non-Patent Document 2: Takao Murakami, Constructions and Chemicals of Natural Originated Substance; Hirokawa Shoten, Sept. 1994: 155-185).

In flowers of plants, anthocyanidins are biosynthesized from naringenin, which is a flavanone as a starting material. Specifically, it has been known that first, by the action of flavonoid 3'-hydroxylase, F3', 5'H or F3'H, naringenin is enzymatically converted into eriodictyol wherein one more hydroxyl group is bonded to B-ring of the flavanone skeleton by the action of flavonoid 3'-hydroxylase, F3',5'H or F3'H, and futher into pentahydroxyflavanone wherein two more hydroxyl groups are bonded thereto. It has also been known that naringenin which is a starting material undergoes the action of flavonoid 3-hydroxylase, F3H, to enzymically converted into dihydrokaempferol, which serves as a substrate, and then enzymatically converted into dihydroquercetin and further into dihydromyricetin by the action of anthocyanidin synthase, AS, wherein one more hydroxyl group and two more hydroxyl groups are bonded to the B-ring, respectively. It has been known that these three dihydrokaempferol, dihydroquercetin, and dihydromyricetin undergo the action of dihydroflavonol reductase, DFR, and of anthocyanidin synthase, AS to be enzymatically converted into pelargonidin, Pgn, cyanidin, Cyn, and delphinidin, Dpn, respectively (Non-Patent Document 2).

In anthocyanidins, the color exhibition is differed depending upon the position of a hydroxyl group or hydroxyl groups substituted on B ring, which is different. For example, in the chemical structure of flower pigment, one which possesses one hydroxyl group on the B ring at 4' position is pelargonidin (Pgn), which exhibits orange to cinnabar red, one which possesses two hydroxyl groups on the B ring at 3'- and 4'-positions is cyaniding (Cyn), which exhibits red to deep red, and one which possesses three hydroxyl groups on the B ring at 3' -, 4' - and 5'-positions is delphinidin (Dpn), which exhibits red purple to purple. Co-existence of them exhibits various flower colors (Non-Patent Document 3: Gendai Kagaku, May 1998, 25-32, Honda Toshio et. al.).

In addition, many anthocyanins have been reported in which various acylated groups are bonded, and it has been recognized that flower colors are determined due to a phenomenon that they are mutually stacked within a molecular to modulate the flower color (intermolecular stacking); they are stacked with other flavonoid glycosides in a sandwich state to modulate (blue) the flower color (bluing) (intermolecular copigmentation); a phenomenon that they are bonded to metal atoms to modulate (blue) the flower color (bluing) (metal-complexation); a phenomenon that acylated groups in a molecule are stacked within the molecule to modulate (blue) the flower color (bluing) (intramolecular copigmentation); a phenomenon that a pH value within a cellar vacuole is changed and other phenomena (Non-Patent Document 4; 4, Goto, T. et al.:Angew. Chem. Int. Ed. Engl., 30:17-33, 1991).

Many of flower pigments have been reported considering flower color (red, blue, yellow, purple, and the like) itself as a genotype (Non-Patent Document 5; Itsuki Yasuyda, Kashoku no Seiri Seikagaku (Physiology and Biochemistry of Flower Color), Uchida Rokakudan; March 1993, p 219-272). In recent years, analysis flower pigments concerning flavonoid pigments has been made which is based on one gene-one enzyme theory proposed by Beale, 1945. One example, which can be mentioned, is a method wherein a genotype is consumed by expressing enzyme systems, dihydroflavonol reactase (DFR) and anthocyanidin synthase (AS), in the anthocyanidin biosynthesis of flower petal of geranium (Pelargonium x hortorum) as E₁/e₁ and E₂/e₂, respectively (Non-Patent Document 6: Kana Kobayashi, Ikusyu Gakkai-Zasshi, 48:169-176,1998). Also, in azalea, a method has been disclosed wherein enzyme systems are assumed as genotypes at the flavonoid biosynthesis precursor in flower petal, but the method is not applicable to flowers except for azalea (Non-Patent Document 7: Heursel, J. and Horn, W.: Z. Pflanzenzuditg, 79: 238-249,1977).

It has been reported that in flowers of petunia, two genes, Ht₁ and Ht₂, control flavonoid 3'-hydroxylase, (F3'H), and two genes, Hf₁, Hf₂, control flavonoid 3',5'-hydroxylase, (F3',5'H) (Non-Patent Document 8: Holton, T. A. et al.: The Plant Cell, 7:1071-1083, 1995).

It has been disclosed that in flowers of petunia, hydroxylation of B-ring of flavonoid 3'-hydroxylase, (F3'H) and flavonoid 3',5'-hydroxylase, (F3'5'H) are two-gene dominated (Non-Patent Document 9: Holton, T. A. et al.: Nature, 366: 276-279, 1993). The crossing method based on flower pigment genotype according to the present invention is characterized in that the flower color is controlled by five multiple allele dominated by one gene, and flower pigment inheritance of flower was not identified as two-gene predominant.

Furthermore, the fact has been clarified that two gene loci contribute to hydroxylation in the flowers of petunia at a gene level (Ht₁, and Ht₂ contribute to hydroxylation at 3' -position of the flavonoid B-ring, and Hf₁, and Hf₂ contribute hydroxylation at 5'-position of the flavonoid B-ring), but there leaves a problem that what type of flower color is inherited to the future generation as pigment genotype does not necessarily have correlation between the pigment genotype and the inheritance of flower color (Non-Patent Document 10: Griesbach, R. J.:J.Heredit., 87:241-245,1996).

Flower color is sensitized by human eyes by entering light to the surface of flower petal, and reflecting light which is not absorbed by pigments existing in epidermal cell of the flower petal. However, since there is difference in sensitivity to light or chroma among individuals, a method for clearly expressing flower color should be required (Non-Patent Document 11: Voss,D.H.:HortSci., 27:1256-1260,1992).

The measurement of flower color utilizing CIELab color coordinate system through a colorimeter has become mainstream. In this method, three color attributes, i.e., hue. Lightness, and chroma (or brightness) are considered as three dimensional global color chart, i.e., three-dimensional color, and the hue difference in the space correctly reflects the difference in the color sensitized by naked eye (Non-Patent Document 12: Gonnet, J. F.: Food Chem., 63:409-415,1998). Consequently, it has been reported that when the flower color is measured to obtain relation between the flower color and endogenous pigment in epidermal cell such as flower fetal, the relation with flower color can be much more correctly obtained (Non-Patent Document 13: Hashimoto, F. et al.:J. Jpn. Soc. Hort. Sci., 69:428-434, 2000; and Non-Patent Document 14: Hashimoto, F. et al.: Biosci. Biotechnol. Biochem., 66: 1652-1659, 2002).

Japanese Unexamined Patent Publication No. 5-184370 (hereinafter referred to as Patent Document 1) discloses a gene of flavonoid hydroxylase (from paragraph [0001] to [0002] of Patent Document 1). Also disclosed therein is "DNA chain or given fragment of DNA chain which codes protein having flavonoid 3',5'-hydroxylase activity is provided. Introduction of this DNA chain into a target plant makes it possible to provide a new cultivar having new color. The present invention also relates to a recombinant vector containing the above DNA chain or a give DNA fragment thereof" (see paragraph [0004] of Patent Document 1).

Japanese Unexamined Patent Publication No. 11-113184 (hereinafter referred to as Patent Document 2) discloses a gene of flavonoid glycosidase (see paragraph [0001] to [0008]). Patent Document 2 discloses that UDP-glucose : a gene of flavonoid 3',5'-O-glycoside transferase is isolated from the flower petal of gentian and it is succeeded to determined the sequence thereof, and that glycosyl transferase which can glycosidize two positions, 3- and 5-position amongst gentiodelphin biosynthesis gene (Paragraph [0005] of Patent Document 2).

Japanese Unexamined Patent Publication No. 11-509733 (hereinafter referred to as Patent Document 3) discloses a composition and process for regulating expression of gene in a plant in Claims 1 to 15.

According to abstract of lecture of 26th International Horticultural Congress (Toronto, Canada), three main antocyanidin genes in flower petal of lisianthus (Eustoma grandiflorum) (Non-Patent Document 15: Uddin,A.F.M.J.et al.:the XXVIth International Horticultural Congress and Exhibition,August 11-17:475 - 476, 2002). We have applied the contents disclosed therein as Japanese Patent Application No. 2003-026598 (hereinafter referred to as Patent Document 4) entitled "Method for Crossing lisianthus based on genotype of its flower pigment" (paragraphs [0001] to [0019] of Patent Document 4). Patent Document 4 discloses that "considering inheritance of three antocyanidins which are main flower pigments of lisianthus, pelargonidin (Pgn), cyaniding (Cyn), and delphinidin (Dpn), examinations have been made by performing self-pollination and cross-pollination, and as a result, new law of inheritance has been found from separation of pigment phenotype of F₁ to F₃-progenies", and that "four multiple allele, H^{T}, H^{F}, H^{D}, and H^{O}, exist in the enzymatic reaction systems of flavonoid 3'-hydroxylase (F3'H) and flavonoid 3',5'-hydroxylase, (F3',5'H) contributing hydroxylation of B ring of pigment precursor, and they control hydroxylation at 3'-position, 5'-position, 3',5'-position, and 3'- and 5'-positions".

US Patent No. 6080920 (hereinafter referred to as Patent Document 5) discloses plants with altered flower and process for providing the same. More specifically, Patent Document 5 discloses that "a novel method for providing transgenic plants, and allows for altered flower color. More specifically, the invention relates to a process for providing transgenic carnation plants, which can exhibit flower colors that do not exist in natural carnation plants".

DE19918365 (hereinafter referred to as Patent Document 6) discloses a method for providing transgenic plants with altered flower color utilizing a nucleic acid which codes flavone synthase II (FNSII). Patent Document 6 discloses that "a nucleic acid which codes flavone synthase II (FNSII), that is (i) one having 1697 base pair (bp) sequence (1) or fragments thereof; (ii) one whose sequence is hybridized with (1) or which has at least 40% of homology thereto, and which codes a protein or polypeptide having FNSII activity; or (iii) a nucleic acid which is genetic equivalent to (i) or (ii)", that "(5) the present invention relates to transgenic plants including (I) or (Ia), and to a method for providing transgenic plants where altered flower color occurs".

Whole of what is multiple allele should be further clear. In addition, although, existence of four multiple alleles has been clear, there is no description about the relation with inherited flower color. Consequently, it should be clear that all of the existing multiple alleles and the relation with flower color.

However, the breeding method based on genotype of flower color itself has many unclear portion concerning separation of the progeny flower color, and thus leaves a large number of problems for practical application. Also, the inheritance of flower pigment of geranium (Pelargonium x hortorum) expressed as E₁/e₁ and E₂/e₂ as disclosed in Non-patent Document 6 is questionable in terms of separation ratio of progeny and, thus, has not yet been put into practice. With regard to Patent Documents, there are problems in terms of the fact that recombination of gene, mutation, for example, with irradiation are required to create new flower color.

It is difficult to expect what kind of flower color does inherited individual has, and the flower color thereof is unclear color determined by naked eye, which is problematic. Also, there are problems in terms of the fact that whether or not the method applicable to lisianthus is applicable to all flowering plants, and that it is not sufficient for correctly measuring flower color and evaluating the same utilizing CIELab color coordinate system to inherit the flowering plant.

The present invention is to clarify the inheritance of biosynthesis of flower pigment, to correctly and numerically measure flower color utilizing a CIELab color coordinate system or such to thereby clarify the relation with flower pigment genotype, and to provide a practical method for crossing plants based on flower pigment genotype for breeding new flowering plants having a new flower color.

All Non-patent Documents and Patent Documents described above (i.e., Non-Patent Documents 1-15 and Patent Documents 1-6) are incorporated herein by reference.

### SUMMARY OF THE INVENTION

In order to solve the problems described above, taking account of inheritance of flavonoid 3'-hydroxylase (F3'H), flavonoid 3',5'-hydroxylase (F3',5'H) and the like, we have discovered new laws, as a result of examination of the separation of inheritance, dihydroflavonol, that inheritance of enzyme system of dihydroflavonol reductase (DFR) and anthocyanidin synthase (AS), participating in biosynthesis of pelargonidin (Pgn), cyanidin (Cyn), and delphinidin (Dpn) are controlled by gene of Pg/pg, Cy/cy, Dp/dp, respectively, and the inheritance of flavonoid 3'-hydroxylase (F3'H), and flavonoid 3',5'-hydroxylase (F3',5'H) is controlled by five multiple alleles. As a result, flower color can be freely created from the pigment genotype of flowering plants without using gene recombinant, radiant ray irradiation or such.

Specifically, the present invention is based on the discovery of new inheritance law from flower pigment phenotypes of main flower pigments F₁ to F₄ generations that as a result of examination of self-pollination and cross pollination taking account of inheritance of three anthocyanidins, pelargonidin (Pgn), cyanidin (Cyn), and delphinidin (Dpn), which are main flower pigments. Also, with regard to Pgn phenotype and Dpn phenotype, it has been discovered that Pgn and Dpn pigments are not co-existing, but they are existing as sole types, or they are inherited together with Cyn pigment. As a result of separation of progenies and chi-square test, existence of gene loci represented as Pg/pg, Cy/cy, Dp/dp in the inheritance of Pgn and Cyn and Dpn pigments has been found at a level of anthocyanin biosynthesis in the flavonoid biosynthesis.

Furthermore, it has been found that there are five multiple alleles, H^{T}, H^{F}, H^{D}, H^{Z}, H^{O}, in the enzymatic reaction of flavonoid 3'-hydroxylase (F3'H) and flavonoid 3',5'-hydroxylase (F3',5'H) participating in hydroxylation of B-ring of pigment precursor, they control hydroxylation at 3'-position, hydroxylation at 5'-position, hydroxylation of 3',5'-positions, hydroxylation at 3'- and 5'-positions, and hydroxylation of 5'-, and 3',5'-position, respectively, and their combination determine the pigment phenotype and flower color phnotye, achieving the present invention.

A method for crossing flowering plants based on their pigment genotypes according to the present invention is creating new flower color utilizing new genotype H^{X}H^{X}·Pg/pg· Cy/cy·Dp/dp, which is heredity of pelargonidin (Pgn), cyanidin (Cyn), and delphinidin (Dpn), which are main flower pigments concerning the flower color expression.

The method for crossing flowering plants based on their pigment genotypes according to the present invention is applicable to flowering plants whose flower color, fruit color, leaf color are inherited in the course of the flavonoid biosynthesis. Specifically, the present invention concerns a method for crossing flowering plants based on their pigment genotypes which creates new flower color utilizing genotype D/d · E/e · H^{X}H^{X} · Pg/pg · Cy/cy · Dp/dp , which is inheritance of pelargonidin (Pgn), cyanidin (Cyn), and delphinidin (Dpn), which are main flower pigments concerning the flower color expression and which is inheritance of double flower type, or edge colored type. With regard to inheritance of pelargonidin (Pgn), cyanidin (Cyn), and delphinidin (Dpn), which are main flower pigments concerning the flower color expression, gene loci of Pgn, Cyn, and Dpn are expressed as Pg/pg, Cy/cy, Dp/dp, respectively, genotypes of flavonoid 3'-hydroxylase (F3'H) and flavonoid 3',5'-hydroxylase (F3',5'H) parcipitating in hydroxylation of B-ring of pigment precursor are expressed as five multiple alleles, H^{T},H^{F},H^{D},H^{Z},H^{O}, two are selected among symbols Pg/pg (one is selected from the combination of PgPg, Pgpg, and pgpg), two are selected among symbols Cy/cy (one is selected from the combination of Cycy, Cycy, and cycy), two are slected among symbols Dp/dp (one is selected from the combination of DpDp, Dpdp, and dpdp), and two are slected amoung symbol H^{T}, H^{F}, H^{D}, H^{Z}, and H^{O} (one is selected from the combination of H^{T}H^{T}, H^{T}H^{F}, H^{T}H^{D}, H^{T}H^{Z}, H^{T}H^{O}, H^{F}H^{F}, H^{D}H^{F}, H^{Z}H^{F}, H^{O}H^{F}, H^{D}H^{D}, H^{D}H^{Z}, H^{D}H^{O}, H^{Z}H^{Z}, H^{Z}H^{O}, and H^{O}H^{O}). Specifically the present invention concerns a method for crossing flowering plants based on their pigment genotypes, which creates new color utilizing a genotype D/d·E/e·H^{X}H^{X}·Pg/pg·Cy/cy·Dp/dp.

In the method for crossing flowering plants based on their pigment genotypes according to the present invention, the flower pigment genotype precipitates in and inherits flavonoid biosynthesis and has a route formula (I): 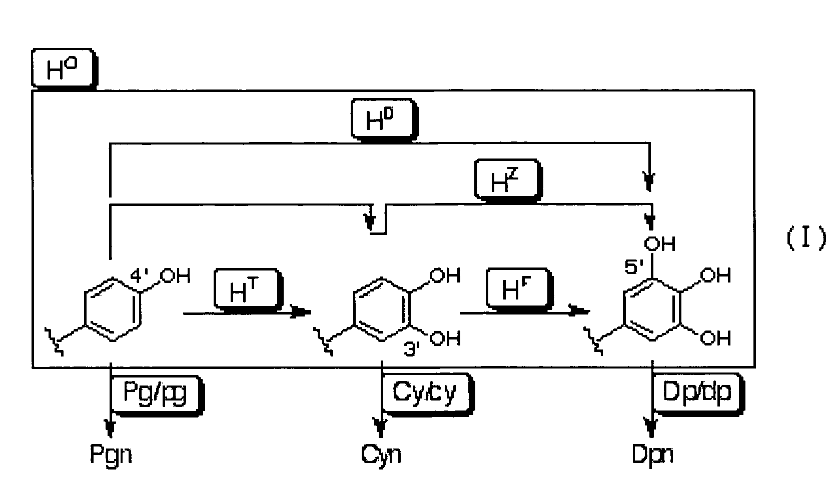
wherein H^{T}, H^{F}, H^{D}, H^{Z}, and H^{O} are multiple alleles participating in hydroxylation of B-ring of flavonoid biosynthesis precursor participating in biosynthesis of pelargonidin (Pgn), cyanidin (Cyn), and delphinidin (Dpn). These five multiple alleles, H^{T}, H^{F}, H^{D}, H^{Z}, and H^{O}, control hydroxylation at 3'-position, hydroxylation at 5'-position, hydroxylation of 3',5'-positions, hydroxylation at 3'- and 5'-positions, and hydroxylation of 5'-, and 3',5'-position; the expression of these five multiple alleles may be other expression method, for example, T, F, D, Z, O; the expression Pg/pg,Cy/cy and Dp/dp means the existence of gene loci corresponding to the expression of dihydroflavonol reductase (DFR) or anthocyanidin synthase (AS) participating in biosynthesis of Pgn, Cyn, and Dpn; D/d is a corolla character of double flower type, and E/e is a corolla character of edge colored).

In the method for crossing flowering plants based on their pigment genotypes according to the present invention, flower color is maternally inherited. More specifically, in the method for crossing flowering plants based on their pigment genotypes according to the present invention, the flower color of flowering plants is maternally inherited, new flower color is created utilizing genotype D/d·E/e·H^{X}H^{X}·Pg/pg·Cy/cy·Dp/dp, which concerns inheritance of pelargonidin (Pgn), cyanidin (Cyn), and delphinidin (Dpn), which are main anthocyanidins concerning flower color expression of flowering plants and inheritance of double flower type and edge colored type concerning flower shape.

A quick reference cap guide according to the present invention is one which determine the combination of crossing plants based on flower pigment genotype for creating a flower color, which displays the combination of multiple allele as described above taking gametes of pollen parents as a row and gametes of seed parents as a line.

A quick reference cap guide according to the present invention is one which displays the pigment phenotype corresponding to the combination of multiple allele.

A quick reference cap guide according to the present invention is one which determines the flower color from the combination of the crossing plants based on the flower genotype, and which displays flower color from the combination of the multiple alleles described above taking gametes of pollen parents as a row and gametes of seed parents as a line.

The terms flowering plants intended herein means flowers, fruits, seeds, leaves containing flavonoids, i.e., Angiospermae of Anthophyta having flower petals, calyxes, bracts, outer perianths, rinds, seed coats, petiolates and the like, and concerns Dicotyledoneae and Monocotyledoneae.

Examples of flowering plants belonging to Sympetalae of Dicotyledoneae include, but are not restricted to Campanulatae, Compositae, Stylidiaceae, Goodeniaceae, Campanulacae, Cucurbitales, Cucurbitaceae, Rubiales, Dipsacaceae, Valerianaceae, Caprifoliaceae, Tubiflorae, Acanthaceae, Lentibulariaceae, Gesneriaceae, Martyniaceae, Pedaliaceae, Bignoniaceae, Scrophulariaceae, Solanaceae, Labiatae, Verbenaceae, Boraginaceae, Hydrophyllaceae, Polemoniaceae, Convolvulaceae, Contortae, Asclepiadaceae, Apocynaceae, Gentianaceae, Loganiaceae, Oleaceae, Plumbaginales, Plumbaginaceae, Primulales, Primulaceae, Myrsinaceae, Ericales, Ericaceae, Pyrolaceae, Diapensiales, Diapensiaceae, and the like.

Examples of flowering plants belonging to Archichlamydeae of Dicotyledoneae include, but are not restricted to, Myrtiflorae: Onagraceae, Melastomataceae, Myrtacear,Combretaceae, Punicaceae, Lythraceae, Elaegnaceae, Thymelaeaceae; Parietales: Begoniaceae, Passifloraceae, Cistaceae, Violaceae, Camelliaceae, Malvales, Malvacaeae, Elaeocarpaceae; Rhamnales: Vitaceae, Rhamnaceae; Sapindales: Balsaminaceae, Hippocastanaceae, Aceraceae, Celastraceae, Aquifoliaceae, Anacardiaceae; Geraniales: Euphorbiaceae, Polygalaceae, Rutaceae, Linaceae, Geraniaceae, Oxalidaceae; Rosales: Leguminosae, Rosaceae, Hamamelidaceae, Pittosporaceae, Saxifragaceae, Crassulaceae; Sarraceniales: Sarraceniaceae, Nepenthaceae, Droseraceae; Papaverales, Brassicaseae, Capparidaceae, Papaveraceae, Ranunculales, Lauraceae, Berberidaceae, Ranunculaceae, Lardizabalaceae, Nymphaeaceae, Annonaceae, Magnoliaceae; Centrospermae: Caryophyllaceae, Nyctaginaceae; Polygonales: Polygonaceae, Urticales: Moraceae,; Myricales: Myricaceae, and the like.

Examples of flowering plants belonging to Monocotyledoneae include, but are not restricted to Orchidales: Orchidaceae; Scitaminea: Cannaceae. Zingiberaceae, Musaceae; Liliiflorae: Iridaceae, Amaryllidaceae, Liliaceae; Commelinales: Pontederiaceae, Commelinaceae, Bromeliaceae; Arales: Araceae, and the like.

In the method for breeding plants based on genotype of the flower color itself, there are many unclear portions in the separation of flower color of progenies, various problems has remained for putting it into practical use. Also, pelargonium x hortorum pigment inheritance expressed as E₁/e₁ and E₂/e₂ as disclosed in Non-Patent Document 6, questions have remained in separation ratio of progenies, having not yet been put into practice. With regard to Patent Documents, there are problems in terms of the fact that recombination of gene, mutation, for example, with irradiation are required to create new flower color. It is difficult to expect what kind of flower color does inherited individual have, and the flower color thereof is unclear color determined by naked eye, which is problematic. Also, there are problems in terms of the fact that whether or not the method applicable to lisianthus is applicable to all flowering plants, and that it is not sufficient for correctly measuring flower color and evaluating the same utilizing CIELab color coordinate system to inherit the flowering plant. The present invention is to clarify the inheritance of biosynthesis of flower pigment, to correctly and numerically measure flower color utilizing a CIELab color coordinate system or such to thereby clarify the relation with flower pigment genotype, and to provide a practical method for crossing plants based on flower pigment genotype for breeding new flowering plants having a new flower color.

The present invention can make it possible to clarify the pigment genotype. For example, the method for crossing flowering plants based on their pigment genotypes to which genotype: D/d·E/e·H^{X}H^{X}·Pg/pg·Cy/cy·Dp/dp and pigment phenotype of Pgn, Cyn, Dpn are attributed is used, and CIELab color coordinate system of flowering plant is used to correctly measure and evaluate flower color, whereby new excellent flower color can be created.

### BEST MODES FOR CARRYING OUT THE INVENTION

The present invention will now be described in detail.

The method for crossing flowering plants based on flower color genotype according to the present invention is a breeding method which is based on genotype concerning the anthocyanidins and which is expressed as five multiple alleles about hydroxylation of the B-ring of the precursor compounds in the biosynthesis of flavonoid.

In the present invention, with regard to the production of precursor compounds of anthocyanidin biosynthesis, when the combination of multiple alleles is H^{T}H^{F}, H^{T}H^{D}, H^{T}H^{Z} with H^{O}-, six (6) types of precursors having one to three hydroxyl groups on the B-ring (naringenin, eriodictyol, pentahydroxyflavanone, dihydrokaempferol; dihydroquercetin, and dihydromyricetin) are produced. When it is combination with H^{T}H^{T}, four (4) types of precursors having one or two hydroxyl groups on the B-ring (naringenin, eriodictyol, dihydrokaempferol, and dihydroquercetin) are produced. When it is combination with H^{F}H^{F}, two (2) types of precursors having one hydroxyl group on the B-ring (naringenin and dihydrokaempferol) are produced. When it is combination with H^{D}H^{F} and H^{D}H^{D}, two (2) types of precursors having three hydroxyl groups on the B-ring (pentahydroxyflavanone and dihydromyricetin) are produced. Moreover, due to the one locus of Pg/pg, which is at a level of anthocyanin synthase, when recessive homozygote (pgpg) is produced, even if naringenin and dihydrokaempferol are produced as precursor compounds, Pgn is never biosynthesized. When it is combination with H^{Z}H^{F}, four (4) types of precursors having two or three hydroxyl groups on the B-ring (eriodictyol, pentahydroxyflavanone, dihydroquercetin, and dihydromyricetin) are produced.

Specifically, the allele of H^{T} controls biosynthetic transformation from naringenin to eriodictyol and that from dihydrokaempferol to dihydroquercetin, and the allele of H^{F} controls biosynthetic transformation from eriodictyol to pentahydroxyflavanone and that from dihydroquercetin to dihydromyricetin. Consequently, the allele of H^{F} does not perform biosynthetic transformation, unless the precursor compounds, eriodictyol or dihydroquercetin exists. On the other hand, the allele of H^{T}, which controls biosynthetic transformation from naringenin to eriodictyol and that from dihydrokaempferol to dihydroquercetin, is characterized in that the substrate is perfectly transformed into pentahydroxyflavanone or dihydromyricetin. The allele of H^{Z}, which controls biosynthetic transformation from naringenin to pentahydroxyflavanone, and biosynthetic transformation from dihydrokaempferol to dihydromyricetin, is characterized in that the substrate is once transformed perfectly into eriodictyol and dihydroquercetin, and then they are transformed into pentahydroxyflavanone and dihydromyricetin. Consequently, when it is paired with the allele of H^{F}, eriodictyol and dihydroquercetin, which are intermediates, are taken as the substrate, and in the genotype H^{Z}H^{F}, four types of precursors having two or three hydroxyl groups on the B-ring (eriodictyol, pentahydroxyflavanone, dihydroquercetin, and dihydromyricetin) are produced.

Consequently, in the case of , H^{D}H^{D} type, , H^{D}H^{F} type, H^{D}H^{Z} type, H^{Z}H^{F} type and H^{Z}H^{Z} type, even if Pg/pg is dominant genotype (PgPg or Pgpg), Pgn is never produced. The allele of H^{O} controls all of the biosynthetic transformation from naringenin to eriodictyol and pentahydroxyflavanone and that from dihydrokaempherol to dihydroquercetin and dihydromyricetin.

In the present invention, for example, as for the pigment genotype of lisianthus flower petal, PgnCynDpn-phenotype can be obtained from H^{T}H^{F}Pg-CyCyDpDp, H^{T}H^{D}Pg-CycyDpDp, H^{T}H^{Z}Pg-CyCyDpDp, and H^{O}-Pg-CyCyDpDp. Also, CynDpn-phenotype can be obtained from H^{T}H^{D}pgpgCyCyDpDp,H^{T}H^{Z}pgpgCyCyDpDp and H^{O}-pgpgCyCyDpDp. Also, Pgn-phenotype can be obtained from H^{F}H^{F}Pg-CyCyDpDp. Cyn-phenotype can be obtained from H^{T}H^{T}pgpgCyCyDpDp Cyn. Dpn-phenotype can be obtained from H^{D}H^{F}-CyCyDpDp, H^{D}H^{Z}-CyCyDpDp, and H^{D}H^{D}-CyCyDpDp. From H^{F}H^{F}pgpgCyCyDpDp, white flower can be obtained. Also, malvidin (Mv) and petunidin (Pt), which are methylated anthocyanidins are included in Dpn pigment phenotype. Furthermore, peonidin (Pn), which is a methylated anthocyanidin is included in Cyn pigment phenotype. The term "white flower" intended herein indicates a flower containing no anthocyanidin at all. In the present invention, PgnDpn phenotype is not obtained. The expression "-" means is dominantly controlled by one former gene and/or allele, and means that any of gene and/or allele can be used. Also, the expression "-" means that any of gene and/or allele can be used.

In the present invention, for example, as for the pigment genotype of lisianthus flower petal, flowers having red purple, red, purple red, pale red, pink can be obtained from PgnCynDpn-phenotype. Also, flowers having red, deep red, pale red, and pink can be obtained from PgnCyn-phenotype. Flowers having pale purple, purple red, purple and blue purple can be obtained from CynDpn-phenotype. Also, flowes having red, pale red, pink, whitish red, cream, and white can be obtained from Pgn-phenotype. Flowers having red, pale red, pink, and whitey red can be obtained from Cyn-phenotype. Flowers having purple can be obtained from Dpn phenotype. White flowers can be obtained from None type (H^{F}H^{F}pgpgCyCyDpDp genotype).

For example, as for the pigment genotype of sweet pea, Lathyrus odoratus, PgnCyn-phenotype can be obtained from H^{T}H^{T}Pg-CyCyDpDp. CynDpn-phenotype can be obtained from H^{T}H^{F}pgpgCyCyDpDp, H^{T}H^{D}pgpgCyCyDpDp, and H^{O}-pgpgCyCyDpDp. Cyn-phenotype can be obtained from H^{T}H^{T}pgpgCyCyDpDp. Dpn-phenotype can be obtained from H^{D}H^{F}-CyCyDpDp and H^{D}H^{D}-CyCyDpDp. White flower can be obtained from H^{F}H^{F}pgpgCyCyDpDp. The term "white flower" intended herein indicates a flower containing no anthocyanidin at all. In the present invention, PgnDpn phenotype is not obtained. The expression "-" means is dominantly controlled by one former gene and/or allele, and means that any of gene and/or allele can be used. Also, the expression "-" means that any of gene and/or allele can be used.

Also, malvidin (Mv) and petunidin (Pt), which are methylated anthocyanidins are included in Dpn pigment phenotype, and they are included in pigment phenotype of Dpn-pgenotype. Furthermore, peonidin (Pn), which is a methylated anthocyanidin is included in Cyn pigment phenotype, and it is included in pigment phenotype of Cyn-genotype.

In the present invention, as for pigment genotype of azalea flower petal and Rhododendron flower petal, Cyn-phenotype can be obtained from H^{T}H^{T}pgpgCyCyDpDp. CynDpn-phenotype can be obtained from H^{T}H^{F}pgpgCyCyDpDp, H^{T}H^{O}pgpgCyCyDpDp, and H^{O}H^{O}pgpgCyCyDpDp. White flower can be obtained from H^{F}H^{F}pgpgCyCyDpDp. The term "white flower" intended herein indicates a flower containing no anthocyanidin at all. In the present invention, PgnDpn phenotype is not obtained. As a characteristic of pigment genotype of azalea flower petal, gene locus of dihydroflavonol reductase (DFR) or and anthocyanidin synthase (AS) participating in biosynthesis of Pgn-phenotype is recessive homozygote (pgpg) and, thus, Pgn pigment is not produced.

Also, malvidin (Mv) and petunidin (Pt), which are methylated anthocyanidins are included in Dpn pigment phenotype, and they are included in pigment phenotype of Dpn-pgenotype. Furthermore, peonidin (Pn), which is a methylated anthocyanidin, is included in Cyn pigment phenotype, and it. is included in pigment phenotype of Cyn-phenotype.

In the method for crossing flowering plants based on flower color genotype according to the present invention, anthocyanin is extracted from colored portion such as flower petals, calyxes, bracts, outer perianths, rinds, seed coats, petiolates and the like, with 50% aqueous acetic acid solution or 50% methanolic-acetic acid (concentration of acetic acid may be from 10 to 50%, and alternatively from 0.5 to 2N normal hydrochloric acid solution may be used instead of acetic acid), and it is hydrolyzed with hydrochloric acid, after which hydrolysate containing anthocyanidin is analyzed by HPLC (Performance Liquid Chromatography) to determine various anthocyanidins. As for genotype of progenies obtained by repeated self-pollination and cross-pollination, dominant homozygote, dominant heterozygote, or recessive homozygote can be determined to freely create various flower colors based on pigment genotype.

### EXAMPLES

The present invention will now be described by referring to the working example. However, it should be noted that the present invention is not restricted thereto.

### EXAMPLE 1

Flower petals, peels, and leaves were collected, and as for flower petals and calyx and the like, colored portions of full color type or edge colored (including double flower), portions having the same color, and white portions were cut out, and they were correctly weighted. Thereafter, an acidic solvent, such as 0.5 to 2 N hydrochloric acid (0.5-2 N HC1), was added, and then anthocyanins were extracted. The extraction was performed according to literature (Uddin, et al.: J. Jpn. Soc. Hort. Sci., 71:40-47, 2002; Wang, et al.: J. Plant Res., 114:213-221,2001; Naotaka Matsuzoe and 5 others: Engakuzatsu, 68:138-145,1999). The extract was subjected to cotton filtration, and thereafter the filtrate was heated at 100degreeC to carry out hydrolysis to obtain one to six types of anthocyanidin. The hydrolysis was performed according to literature (Uddin, et al.:J. Jpn. Soc. Hort. Sci., 71: 40-47,2002). After the reaction was completed, the reaction mixture was filtrated through a membrane filter, and then analyzed by an HPLC apparatus. The HPLC analysis conditions were according to the method described in literature (Uddin, et al.: J. Jpn. Soc. Hort. Sci., 71: 40-47,2002). From HPLC chromatographic chart, three types of anthocyanidins, i.e., pelargonidin (Pgn), cyanidin (Cyn), delphinidin (Dpn), and three types of methylated anthocyanidins, i.e., peonidin (Pn), petunidin, (Pt), and malvidin (Mv) were calculated as occupied area where total peak area was assumed to be 100%. From the resulting inherent peaks, the pigment genotype of the measured flower was determined as for anthocyanidins.

Flower petals, peels, and leaves were collected, and as for flower petals and calyx and the like, colored portions of full color type or edge colored (including double flower), portions having the same color, and white portions were cut out, and they were measured for flower color utilizing a colorimeter. CIELab color coordinate system was used as a color coordinate system, and measurement conditions and measurement apparatuses were used as disclosed in the method of literature (Wang, et al.: J. Plant Res., 114:33-43,2001).

Three cultivars (Gentianaceae) of lisianthus; Eustoma, Gentianaceae, i.e., Royal Violet (CynDpn-phenotype), Micky Rose (PgnCynDpn-phenotype), and Asuka no Kurenai (PgnCyn-phenotype) were used to examine separation of S₁-generation by self-pollination. The results are shown in Table 1. Similarly. Three cultivars (Gentianaceae) of Royal Violet (CynDpn-phenotype), Micky Rose (PgnCynDpn-phenotype), and Asuka no Kurenai (PgnCyn-phenotype) were used to examine separation of F₁-generation by cross-pollination. The results are shown in Table 2. From the results, pigment genotypes of Royal Violet (CynDpn-phenotype), Micky Rose (PgnCynDpn-phenotype), and Asuka no Kurenai (PgnCyn-phenotype) were determined.

**Table 1**

| Pigment Phenotype of S₁ Anthocyanidin | Observed Value | Pigment Genotype of S₁ Generation | Exp. Value (Sep. Ratio) | X² Detected Value *P<0.05 | Adapted Value |
|---|---|---|---|---|---|
| Self-pollination of Micky Rose (ddeeH^{T}H^{F}pg-CyCyDpDp pigment genotype) (F₁) Total 130 Individuals | | | | | |
| PgnCynDpn | 62 | ddeeH^{T}H^{F}pg- CyCyDpDp | 6 | 11.887 | 0.036 |
| PgnCyn | 28 | ddeeH^{T}H^{T}pg- CyCyDpDp | 3 | | |
| CynDpn | 15 | ddeeH^{T}H^{F}pgpg CyCyDpDp | 2 | | |
| Pgn | 19 | ddeeH^{T}H^{T}pg- CyCyDpDp | 3 | | |
| Cyn | 3 | ddeeH^{T}H^{T}pgpg CyCyDpDp | 1 | | |
| None | 3 | ddeeH^{F}H^{F}pgpg CyCyDpDp | 1 | | |

| Self-pollination of Royal Violet (ddeeH^{O}H^{D}pgpgCyCyDpDp pigment genotype) (F₁) Total 183 Individuals | | | | | |
|---|---|---|---|---|---|
| PgnCyn | 138 | ddeeH^{O}-pgpgCyCyDpDp | 3 | 0.164* | 0.898 |
| Dpn | 45 | ddeeH^{D}H^{D}pgpgCyCyDpDp | 1 | | |

| Self-pollination of Asukanobeni (ddeeH^{T}H^{T}pgpgCyCyDpDp pigment genotype) (F,) Total 142 Individuals | | | | | |
|---|---|---|---|---|---|
| PgnCyn | 142 | ddeeH^{T}H^{T}pgpgCyCyDpDp | 1 | - | 1.000 |
| Table 2 | | | | | |

| Pigment Phenotype of F₁ Anthocyanidin | Observed Value | Pigment Genotype of F₁ Generation | Exp. Value (Sep. Ratio) | X² Detected Value *P<0.05 | Adapted Value |
|---|---|---|---|---|---|
| Cross-pollination of Micky Rose (ddeeH^{T}H^{F}pg-CyCyDpDp) and Royal Violet (ddeeH^{O}H^{D}pgpgCyCyDpDp) :Total 160 Individuals | | | | | |
| PgnCynDpn | 52 | ddeeH^{O}-Pg-CyCyDpDP/ ddeeH^{T}H^{D}Pg-CyCyDpDp | 3 | 1.842* | 0.398 |
| CynDpn | 63 | ddeeH^{O}-pgpgCyCyDpdp/ ddeeH^{T}H^{D}pgpgCyCyDpDp | 3 | | |
| Dpn | 45 | ddeeH^{T}H^{T}pg-CyCyDpDp | 2 | | |

| Cross-pollination of Royal Violet (ddeeH^{O}H^{D}pgpgCyCyDpDp) and Asukanobeni (ddeeH^{T}H^{T}pgpgCyCyDpDp) :Total 167 Individuals | | | | | |
|---|---|---|---|---|---|
| PgnCynDpn | 137 | ddeeH^{O}H^{T}PgpgCyCyDpDP/ ddeeH^{D}H^{T}PgpgCyCyDpDp | 3 | 1.842* | 0.398 |

| Cross-pollination of Asukanobeni (ddeeH^{T}H^{T}pgpgCyCyDpDp) and Micky Rose (ddeeH^{T}H^{F}pg-CyCyDpDp) :Total 208 Individuals | | | | | |
|---|---|---|---|---|---|
| PgnCynDpn | 103 | ddeeH^{T}H^{F}Pg-CyCyDpDP | 1 | 0.019* | 0.890 |
| PgnCyn | 105 | ddeeH^{T}H^{T}Pg-CyCyDpDp | 1 | | |

From Tables 1 and 2, it has been proven that Royal Violet has a pigment genotype of ddeeH^{O}H^{D}pgpgCyCyDpDp, Micky Rose has a pigment genotype of ddeeH^{T}H^{F}PgpgCyCyDpDp, and Asuka no Kurenai has a pigment genotype of ddeeH^{T}H^{T}PgPgCyCyDpDp. Royal Violet has a purple flower color, Micky Rose has red purple flower color, and Asuka No Beni has a red flower color. In Table, 1, none-pigment phenotype means white flower color.

### EXAMPLE 3

S₁-generations of lisianthus shown in Table 1 were used as parent stain, and they were subjected to self-pollination, and separated to obtain S₂-generations, which were examined to determine pigment genotypes of various lines. The results are shown in Table 3.

**Table 3**

| Pigment Phenotype | Observed Value | Pigment Genotype | Exp. Value (Sep. Ratio) | X² Detected Value *P<0.05 | Adapted Value |
|---|---|---|---|---|---|
| Separation of progenies by Self-pollination of G2D3B27E (ddeeH^{T}H^{F}pgpgCyCyDpDp pigment genotype) Line (F₂) | | | | | |
| CynDpn | 22 | ddeeH^{T}H^{F}pgpg CyCyDpDp | 2 | 1.811 * | 0.404 |
| Cyn | 9 | ddeeH^{T}H^{T}pgpg CyCyDpDp | 1 | | |
| None | 6 | ddeeH^{F}H^{F}pgpg CyCyDpDp | 1 | | |

| Separation of progenies by Self-pollination of G2D3B29A (ddeeH^{F}H^{F}PgpgCyCyDpDp pigment genotype) Line (F₂ and F₃) | | | | | |
|---|---|---|---|---|---|
| Pgn | 24 | ddeeH^{F}H^{F}Pg- CyCyDpDp | 3 | 0.771* | 0.380 |
| None | 11 | ddeeH^{F}H^{F}pgpg CyCyDpDp | 1 | | |

| Separation of progenies by Self-pollination of G2D3B25F (ddeeH^{F}H^{F}PgPgCyCyDpDp pigment genotype) Line (F₂ and F₃) | | | | | |
|---|---|---|---|---|---|
| Pgn | 76 | ddeeH^{F}H^{F}PgPg CyCyDpDp | 1 | - | 1.000 |

| Separation of progenies by Self-pollination of G2D3B27Y (ddeeH^{T}H^{T}pgpgCyCyDpDp pigment genotype) Line (F₂) | | | | | |
|---|---|---|---|---|---|
| Cyn | 12 | ddeeH^{T}H^{T}pgpgCyCyDpDp | 1 | - | 1.000 |

| Separation of progenies by Self-pollination of G2D3B26B (ddeeH^{F}H^{F}pgpgCyCyDpDp pigment genotype) Line (F₂ and F₃) | | | | | |
|---|---|---|---|---|---|
| None | 31 | ddeeH^{F}H^{F}pgpgCyCyDpDp | 1 | - | 1.000 |

| Separation of progenies by Self-pollination of J5A2H16B (ddeeH^{O}H^{O}pgpgCyCyDpDp pigment genotype) Line (F₂ and F₃) | | | | | |
|---|---|---|---|---|---|
| CynDpn | 22 | ddeeH^{O}H^{O}pgpgCyCyDpDp | 1 | - | 1.000 |

| Separation of progenies by Self-pollination of J5A2H13CE (ddeeH^{O}H^{D}pgpgCyCyDpDp pigment genotype) Line (F₂ and F₃) | | | | | |
|---|---|---|---|---|---|
| CynDpn | 39 | ddeeH^{O}-pgpgCyCyDpDp | 3 | 0.491 * | 0.484 |
| Dpn | 16 | ddeeH^{D}H^{D}pgpgCyCyDpDp | 1 | | |

| Separation of progenies by Self-pollination of J5A2H110C1A (ddeeH^{D}H^{D}pgpgCyCyDpDp pigment genotype) Line (F₂ and F₃) | | | | | |
|---|---|---|---|---|---|
| Dpn | 24 | ddeeH^{D}H^{D}pgpgCyCyDpDp | 1 | - | 1.000 |

| Separation of progenies by Self-pollination of W1C3B111Y (ddeeH^{T}H^{T}PgPgCyCyDpDp pigment genotype) Line (F₂ and F₃) | | | | | |
|---|---|---|---|---|---|
| Dpn | 24 | (ddeeH^{T}H^{T}PgPgCyCyDpDp | 1 | - | 1.000 |

As is clear from Table 3, G2D3B25F line (white, red white, cream, or pink flowers) only having Pgn-pigment was obtained from ddeeH^{F}H^{F}PgPgCyCyDpDp pigment genotype. G2D3B27Y line (red white or pink flowers) only having Cyn-pigment was obtained from ddeeH^{T}H^{T}pgpgCyCyDpDp pigment genotype. G2D3B26B line (white flower) having no pigment was obtained as non-phenotype from ddeeH^{F}H^{F}pgpgCyCyDpDp pigment genotype. J5A2H16B line (red purple flower) having CynDpn-pigment phenotype was obtained from ddeeH^{O}H^{O}pgpgCyCyDpDp pigment genotype. J5A2H110C1A line (purple flower) having Dpn-pigment phenotype was obtained from ddeeH^{D}H^{D}pgpgCyCyDpDp pigment genotype. W1C3B111Y line (red flower) having PgnCyn-pigment phenotype was obtained from ddeeH^{T}H^{T}PgPgCyCyDpDp pigment genotype. It has been understood that all of them are pure line (dominant or recessive homozygote).

### EXAMPLE 4

When red white flower having Pgn-phenotype (G2D3B25F line, ddeeH^{F}H^{F}PgPgCyCyDpDp pigment genotype) and red flower having Cyn-phenotype (G2D3B27Y line, ddeeH^{T}H^{T}pgpgCyCyDpDp pigment genotype) were subjected to cross-pollination, red purple flower having PgnCynDpn-phenotype (ddeeH^{T}H^{F}PgpgCyCyDpDp pigment genotype) was obtained. Non-Patent Document 6 (Kana Kobayashi, Ilusyu Gakkai-Zasshi, 48:169-176,1998) which assumes pigment phenotype), discloses that flower having PgnCyn-phenotype is obtained, and the separation of red purple flower having PgnCynDpn-phenotype cannot be explained.

### EXAMPLE 5

When red flower having PgnCyn-phenotype (W1C3B111Y line, ddeeH^{T}H^{T}PgPgCyCyDpDp pigment genotype) and white flower (none-phenotype, ddeeH^{F}H^{F}pgpgCyCyDpDp pigment genotype) were subjected to cross-pollination, red purple flower having PgnCynDpn-phenotype was obtained. Non-Patent Document 6 (KanaKobayashi, IlusyuGakkai-Zasshi, 48:169-176,1998) which assumes pigment phenotype), discloses that flower having PgnCyn-phenotype is obtained, and the separation of red purple flower having PgnCynDpn-phenotype cannot be explained.

### EXAMPLE 6

Bridal Violet (edge colored flower, F₁ line) which is cultivar of lisianthus was subjected to self-pollination, its flower color separation was examined. As a result, as shown in Table 4, all of progenies were obtained as edge colored, which was dominant and determined pigment genotype and flower coloration. Line F3I1A2D1D was white flower with edge colored having dominant homozygote.

**Table 4**

| Line | Ind. | Pigment Constitution of Anthocyanidin | | | Pigment Genotype | CIELab color coordinate system | | |
|---|---|---|---|---|---|---|---|---|
| | | Pg(%) | Cy(%) | Dp(%) | | L* | C* | h |
| Self-pollination of F3I1A2D1(X² Detected Value 1.322; Adapted Value 0.723) | | | | | | | | |
| F3I1A2D1A | 35 | - | 5.8 | 94.2 | ddeeH^{Z}H^{F}Pg-CyCyDpDp | 35.5 | 53.2 | -28.1 |
| F3I1A2D1B | 15 | - | - | 100 | ddeeH^{Z}H^{Z}Pg-CyCyDpDp | 34.4 | 53.3 | -38.3 |
| F3I1A2D1C | 9 | 100 | - | - | ddeeH^{F}H^{F}Pg-CyCyDpDp | 74.6 | 18.3 | 13.7 |
| F3I1A2D1D | 3 | - | - | - | ddeeH^{Z}H^{F}pgpgCyCyDpDp | 82.7 | 9.3 | 91.7 |

### EXAMPLE 5A

Seven (7) lines of lisianthus, G4I5A3I1F4(CynDpn-phenotype, red purple flower), A13B1B3I4 (PgnCyn-phenotype, red flower), G2D3B2I5C33(PgnCyn-phenotype, red flower), G2D3B2I5C3A(Cyn-phenotype, red flower), I5A21I3F12 (Dpn-phenotype, purple red flower), G2D3B2I5C36 (Pgn-phenotype, white yellow flowe), and G2D3B2I5C37 (none-phenotype, white flower), were subjected to self-pollination, and separation of 180 progenies was examined. The results are shown in Table 5.

**Table 5**

| Line | Ind. | Pigment Constitution of Anthocyanidin | | | Pigment Genotype | CIELab color coordinate system | | |
|---|---|---|---|---|---|---|---|---|
| | | Pg(%) | Cy(%) | Dp(%) | | L* | C* | h |
| G4I5A3U1F4 | 34 | - | 66.9 | 3.8 | ddeeH^{O}H^{O}pgpgCyCyDpDp | 64.0 | 34.6 | -31.0 |
| A1C3BIB314 | 96 | 92.1 | 7.7 | - | ddeeH^{T}H^{T}PgPgCyCyDpDp | 40.6 | 56.4 | -4.7 |
| G2D3B215C33 | 8 | 98.4 | 1.6 | - | ddeeH^{T}H^{T}pgpgCyCyDpDp | 60.6 | 29.4 | -4.3 |
| G2D3B215C3A | 1 | - | 100 | - | ddeeH^{T}H^{T}PgPgCyCyDpDp | 46.4 | 48.2 | -10.7 |
| I5A2H113F12 | 13 | - | - | 100 | ddeeH^{D}H^{D}pgpgCyCyDpDp | 25.1 | 77.7 | -28.8 |
| G2D3B215C36 | 23 | 100 | - | - | ddeeH^{F}H^{F}PgPgCyCyDpDp | 86.4 | 6.5 | 88.3 |
| G2D3B215C37 | 5 | - | - | - | ddeeH^{F}H^{F}pgpgCyCyDpDp | 87.0 | 10.7 | 114.2 |

As shown in Table 5, these lines had the same pigment constitutions, pigment-genotypes, and flower colors, as those of the parent strains, respectively. The numerical values except for number of individuals in each line are average values relative to the individual in each line. All of the pigment-genotypes were homozygotes. Two lines of G4I5A3I1F4 and I5A21I3F12 having different pigment constitutions and different genotypes had similar hue angles (h), which were -31.0 and -28.8 degree, and the flower colors were red purple. Two lines of A1C3B1B3I4 and G2D3B2I5C33 having different lines gave similar hue angles (h) (-4.7, and -4.3 degree, respectively), and the flower colors were color directed toward red. However, A1C3B1B3I4 line had C* value showing chroma of 56.4, which was approximately twice that of G2D3B2I5C33 lime, having deep red color.

On the other hand, G2D3B2I5C33 line was pale red flower. The hue angle (h) of G2D3B2I5C36 (Pgn-pigment phenotype) indicated 88.3 degree, and had a color directed toward yellow. The C* value thereof was as low as 6.5, indicating small chroma flower. Consequently, in spite of containing anthocyanin in the flower petal, the flower color was confirmed as cream by naked eye. The hue angle of G2D3B2I5C37 line, having white color was 114.2, and had a color directed toward green yellow. The C* value thereof was as low as 10.7 and thus, the flower had a color near white color. However, it was confirmed as pale yellow by naked eye.

### EXAMPLE 6A

Twelve (12) lines, 298 individuals of lisianthus; G2D3B2I5C31(PgnCynDpn-phenotype, red purple flower), G2D3B2I5C32 (PgnCynDpn-phenotype, red flower), G2D3B2I5C34 (PgnCyn-phenotype, red orange flower), G2D3B2I5C35 (Pgn-phenotype), white flower), G2D3B2I5C38 (CynDpn-phenotype, white flower), G2D3B2I5C39 (CynDpn-phenotype, red purple flower), I5A21I3F11 (CynDpn-phenotype, purple red flower), A1C3B1B3IMA (PgnCynDpn-phenotype, red purple flower), A1C3B1B3IMB (PgnCyn-phenotype, red flower), I5A21I3FMA (PgnCynDpn-phenotype, purple flower), I5A21I3FMB (CynDpn-phenotype, purple flower), and I5A21I3FMC (Dpn-phenotype, purple flower) were examined for pigment constitution, pigment-genotype, and flower coloration. The results are shown in Table 6.

**Table 6**

| Line | Ind. | Pigment Constitution of Anthocyanidin | | | Pigment Genotype | CIELab color coordinate system | | |
|---|---|---|---|---|---|---|---|---|
| | | Pg(%) | Cy(%) | Dp(%) | | L* | C* | h |
| G2D3B215C31 | 32 | 26.0 | 67.8 | 3.9 | ddeeH^{T}H^{F}Pg-CyCyDpDp | 51.8 | 37.8 | -18.7 |
| G2D3B215C32 | 5 | 20.5 | 72.5 | 2.6 | ddeeH^{T}H^{F}Pg-CyCyDpDp | 78.8 | 6.5 | 28.6 |
| G2D3B215C34 | 9 | 34.7 | 64.1 | - | ddeeH^{T}H^{T}Pg-CyCyDpDp | 80.4 | 7.8 | 55.8 |
| G2D3B215C35 | 4 | 100 | - | - | ddeeH^{F}H^{F}Pg-CyCyDpDp | 63.3 | 23.5 | 4.4 |
| G2D3B215C38 | 4 | - | 82.3 | 15.6 | ddeeH^{D}H^{D}pgpgCyCyDpDp | 84.6 | 8.5 | 97.5 |
| G2D3B215C39 | 7 | - | 79.7 | 17.7 | ddeeH^{T}H^{F}pgpgCyCyDpDp | 54.0 | 34.6 | -22.1 |
| 15A2H113F11 | 31 | - | 7.6 | 90.7 | ddeeH^{O}H^{D}pgpgCyCyDpDp/ ddeeH^{O}H^{O}pgpgCyCyDpDp | 25.0 | 76.3 | -28.0 |
| AIC3B1B31MA | 46 | 38.2 | 67.3 | 2.8 | ddeeH^{T}H^{F}Pg-CyCyDpDp | 53.5 | 45.4 | -18.4 |
| AlC3BlB31MB | 46 | 88.6 | 11.4 | - | ddeeH^{T}H^{D}Pg-CyCyDpDp | 66.7 | 32.5 | -8.0 |
| I5A2Hll3FMA | 42 | 1.3 | 11.7 | 85.5 | ddeeH^{T}H^{D}Pg-CyCyDpDp/ ddeeH^{O}H^{X}Pg-CyCyDpDp | 30.0 | 75.2 | -32.0 |
| I5A2Hll3FMB | 43 | - | 8.4 | 90.5 | ddeeH^{T}H^{D}pgpgCyCyDpDp/ ddeeH^{O}H^{X}pgpgCyCyDpDp | 30.3 | 75.2 | -32.5 |
| I5A2Hll3FMC | 39 | - | - | 100 | ddeeH^{D}H^{F}--CyCyDpDp | 32.4 | 71.6 | -34.0 |

As shown in Table 6, pigment phenotypes of all lines were hetero types. The numerical values except for number of individuals in each line are average values relative to the individual in each line. The two lines of G2D3B2I5C31 (PgnCynDp-pigment phenotype, red purple flower) and A1C3B1B3IMA (PgnCynDpn-pigment phenotype, red purple flower) had similar values of anthocyanidin pigment constitution and pigment genotype. On the other hand, The line of G2D3B2I5C32 (PgnCynDpn-pigment phenotype, red flower) has similar pigment genotype and pigment constitution to those of G2D3B2I5C31, and A1C3B1B3IMA, but its hue angle was 28.6 degree indicating a color directed toward red orange. However, from the fact that the C* value thereof was as low as 6.5, the flower color confirmed by naked eye was pale reddish white flower. Two lines of G2D3B2I5C34 and A1C3B1B3IMB having the same pigment genotype had quite different pigment constitution and flower coloration. Specifically, the hue angle of G2D3B2I5C34 was 55. 8 degree and it had a color directed toward orange (orange flower near white by naked eye), while the hue angle of A1C3B1B3IMB was -8.0 degree and it had a color directed toward red (red flower by naked eye). Two lines of G2D3B2I5C38 and G2D3B2I5C39 having the same pigment genotype had quite different pigment constitution and flower coloration. Specifically, the hue angle of G2D3B2I5C38 was 97.5 degree and it has a color directed toward yellow (yellow flower near white by naked eye), but the hue angle of G2D3B2I5C39 was -22.1 and it had a color directed toward red (red flower by naked eye). Four lines of I5A2H1I3F11, I5A2H1I3FMA, I5A2H1I3FMB, and I5A2H1I3FMC having mutiple alleles H^{O} or H^{D} had hue angles lower than -20 degree, they had a color directed toward purple red, and had purple flower by naked eye.

### EXAMPLE 7

A method for creating an F₁ seed of lisianthus will now be described in detail.

Single whole pink flower having Cyn pigment phenotype (ddeeH^{T}H^{T}pgpgCyCyDpDp pigment genotype, homozygote) and single whole white flower having Pgn pigment phenotype (ddeeH^{F}H^{F}PgPgCyCyDpDp pigment genotype, homozygote) were crossed to obtain singe whole red purple flower having PgnCynDpn pigment phenotype (ddeeH^{T}H^{F}PgpgCyCyDpDp pigment genotype, hetero).

Single whole red purple flower having CynDpn pigment phenotype (ddeeH^{O}H^{O}pgpgCyCyDpDp pigment genotype, homozygote) and single whole pink flower having Pgn pigment phenotype (ddeeH^{F}H^{F}PgPgCyCyDpDp pigment genotype, homozygote) were crossed to obtain singe whole red purple flower having PgnCynDpn pigment phenotype (ddeeH^{O}H^{F}PgpgCyCyDpDp pigment genotype, hetero).

Single whole red flower having PgnCyn pigment phenotype (ddeeH^{T}H^{T}PgPgCyCyDpDp pigment genotype, homozygote) and single whole pink flower having Pgn pigment phenotype (ddeeH^{F}H^{F}PgPgCyCyDpDp pigment genotype, homozygote) were crossed to obtain singe whole pink intermediate colored flower having PgnCynDpn pigment phenotype (ddeeH^{T}H^{F}PgpgCyCyDpDp pigment genotype, hetero).

According to a plan that G2D3B2I5C36 (Pgn pigment phenotype, ddeeH^{F}H^{F}PgPgCyCyDpDp pigment, white yellow flower) of lisianthus and G4I5A3I1F4(CynDpn pigment phenotype, ddeeH^{O}H^{O}pgpgCyCyDpDp pigment genotype, red purple flower )of lisianthus (see Table 5) were subjected to cross-pollination to obtain red flower, the crossing was performed. The crossing gave G2D3G4I5 line (16 individuals) was obtained as flower of F₁ seed. All of the phenotypes were PgnCynDpn (Pg: 24.7%, Cy: 72.4%, Dp: 2.9%), and had ddeeH^{O}H^{F}PgpgCyCyDpDp pigment genotype. The flower color confirmed by naked eye was red purple. As for flower coloration, the hue angle (h) was -18.5 degree, and it has a color directed toward red purple. Brightness L * value was 61.9 which was bright similar to that of G4I5A3I1F4 line, and the hue C* value was 40.7, which was slightly bright red purple flower. Consequently, Pgn pigment phenotype free of G4I5A3I1F4 could be incorporated therein by utilizing G2D3B2I5C36 line making it possible to create more red flower than G4I5A3I1F4 line, as planed.

### EXAMPLE 8

A1C3B1B3I line having PgnCyn pigment genotype (pigment genotype: ddeeH^{T}H^{T}PgPgCyCyDpDp) of lisianthus and I5A2H1I3F having CynDpn pigment genotype (pigment genotype: ddeeH^{O}H^{D}pgpgCyCyDpDp) were used to examine separation of progenies by cross- pollination. The results are shown in Table 7. When the crossing was made using A1C3B1B3I line as a seed parent and I5A2H1I3F as pollen parent, A1C3B1B3IRA line and A1C3B1B3IRB line were obtained in a ratio of 1:1 to determine their pigment genotype and flower coloration. A1C3B1B3IRA line mainly contains Pgn pigment, and the hue angle thereof was -8.0 degree, and flower color was in the direction toward pale red. The C* value indicating brightness was 33.3, and it was palered flower. A1C3B1B3IRB line mainly contained Cyn pigment, the hue angle was -18.9 degree, and flower color was in the direction toward purplish red. The C* value indicating brightness was 47.1, and it was red flower.

On the other hand, when the crossing was made using I5A2H1I3F as seed parent and A1C3B1B3I as pollen parent, 79 indivudials of I5A2H1I3FAS were obtained, and thir pigment genotype and flower coloration were determined. The hue angle was -30.2 degree, and the flower coloration was directed toward purple red.

**Table 7**

| Line | Pigment Constitution of Anthocyanidin | | | | Pigment Genotype | CIELab color coordinate system | | |
|---|---|---|---|---|---|---|---|---|
| | Ind. | Pg(%) | Cy(%) | Dp(%) | | L* | C* | h |
| A1C3B1B31 | 1 | 95.0 | 5.0 | - | ddeeH^{T}H^{T}PgPgCyCyDpDp | 65.2 | 29.5 | -5.3 |
| I5A2H113F | 1 | - | 4.0 | 96.0 | ddeeH^{O}H^{D}pgpgCyCyDpDp | 24.6 | 75.7 | -29.4 |

| A1C3B1B31 (Seed Parent) x I5A2H113F (Pollen Parent) | | | | | | | | |
|---|---|---|---|---|---|---|---|---|
| A1C3B1B31RA | 29 | 91.6 | 6.0 | 2.4 | ddeeH^{T}H^{D}PgpgCyCyDpDp | 65.8 | 33.3 | -8.0 |
| A1C3B1B31RB | 29 | 33.3 | 64.6 | 2.1 | ddeeH^{T}H^{O}gpgCyCyDpDp | 52.2 | 47.1 | -18.9 |

| I5A2H113F (Seed Parent) x A1C3B1B31 (Pollen Parent) | | | | | | | | |
|---|---|---|---|---|---|---|---|---|
| 15A2H113FAS | 79 | 0.5 | 9.6 | 89.8 | ddeeH^{D}H^{T}PgpgCyCyDpDp/ ddeeH^{O}H^{T}PgpgCyCyDpDp | 27.4 | 76.3 | -30.2 |

It has been proven from the results of Table 7 that pigment biosynthesis between A1C3B1B3I line and I5A2H1I3F line is nuclear inherited, and there is flower color inheritance due to inheritance due to cytoplasmic (particularly maternal inheritance) between them. By the use of this inheritance, flower coloration near mother strain could be created without loosing pigment nuclear genotype.

### EXAMPLE 9

A4B3F2K2 line (F₂, double flower) G2D3B2I59A line (F₃), G4H5G2D39A line (F₂) of lisianthus were subjected to self-pollination. The results are shown in Table 8. In A4B3F2K2 line (F₂), pigment genotype was homozygote, and flower coloration was separated at a ratio of approximately 3;1. From G2D3B2I59A(F₃) line and G4H5G2D39A(F₂) line, the flower coloration was separated according to pigment genotype. As a result, it can be proven that different flower colaration is separated from the same genotype as in A4B3F2K2(F₂) self-pollination line.

**Table 8**

| Line | Ind. | Pigment Constitution of Anthocyanidin | | | Pigment Genotype | CIELab color coordinate system | | |
|---|---|---|---|---|---|---|---|---|
| | | Pg(%) | Cy(%) | Dp(%) | | L* | C* | h |
| Self-pollination of A4B3F2K2 (F₂) (X² Detected Value: 2.564, Adapted Value: 0.109) | | | | | | | | |
| A4B3F2K21 | 34 | 100 | - | - | DDeeH^{T}H^{T}pgpgCyCyDpDp | 68.7 | 33.6 | -6.2 |
| A4B3F2K22 | 18 | 100 | - | - | DDeeH^{T}H^{T}pgpgCyCyDpDp | 88.8 | 7.1 | 14.5 |

| Self-pollination of G2D3B2159A (F₃) (X² Detected Value: 0.961, Adapted Value: 0.327) | | | | | | | | |
|---|---|---|---|---|---|---|---|---|
| G2D3B2159A1 | 11 | 100 | - | - | ddeeH^{F}H^{F}Pg-CyCyDpDp | 88.1 | 8.1 | 98.4 |
| G2D3B2159A2 | 6 | - | - | - | ddeeH^{F}H^{F}pgpgCyCyDpDp | 89.3 | 8.6 | 105.2 |

| Self-pollination of G45H5G2D39A (F₂) (X² Detected Value: 12.18, Adapted Value: 0.0005) | | | | | | | | |
|---|---|---|---|---|---|---|---|---|
| G45H5G2D39A1 | 10 | 18.3 | 75.2 | 6.5 | ddeeH^{O}H^{O}pg-CyCyDpDp | 63.0 | 39.4 | -20.1 |
| G45H5G2D39A2 | 13 | | | | ddeeH^{O}H^{O}pgpgCyCyDpDp | 71.8 | 28.3 | -26.2 |

### EXAMPLE 10

Double flower (multi-petal) lisianthus was subjected to self-pollination to separate 84 individuals of double flower and 30 individuals of single flower at a ratio of to 3:1. As a result, genotype of double flower may be indicated as D/d. D/d means the first letter of double flower, and represents dominant/recessive. Indication is the same even if another first letter is used. Consequently, from DD and Dd genotypes, double flower was obtained, and from dd genotype, single flower was obtained. In double flower, there are rose double flower and frill double flower, they were obtained from genotypes Dᵣ/d, and D_{f}/d, respectively.

### EXAMPLE 11

Lisianthus with edge colored (only edge of flower petal is colored) was subjected to self-pollination to separate 229 individuals of edge colored flowers and 77 individuals of whole colored flowers at a ratio of 3:1. As a result, genotype of edge colored) can be indicated as E/e. E/e means the first letter of edge colored, and represents dominant/recessive. Indication is the same even if another first letter is used. Consequently, from EE and Ee genotypes, edge colored flower was obtained, and from ee genotype, whole colored flower was obtained.

### EXAMPLE 12

Flower petal pigment of sweet pea (Leguminosae) was analyzed to examine pigment genotypes of flower petal of various lines. As a result, as shown in Table 9, pigment genotypes of flower petal of various lines were clarified. Malvidin (Mv) and petunidin (Pt), which are methylated anthocyanidins, were contained in Dpn pigment phenotype, and they were included in pigment genotype which produces Dpn. Moreover, in Cyn pigment phenotype, peonidin (Pn), which is methylated anthocyanidin, was contained, and it was included in pigment genotype which produces Cyn.

**Table 9**

| Line | Ind. | Pigment Constitution of Anthocyanidin | | | | | | Pigment Genotype |
|---|---|---|---|---|---|---|---|---|
| | | Pg(%) | Cy(%) | Pn(%) | Dp(%) | Pt(%) | Mv(%) | |
| Purple Type | 5 | - | - | - | 5.0 | 18.4 | 76.6 | ddeeH^{D}H^{D}--CyCyDpDp |
| Blue Purple | 7 | - | 4.4 | 1.4 | 24.9 | 21.9 | 47.4 | ddeeH^{T}H^{D}pgpgCyCyDpDp |

| Type | | | | | | | | |
|---|---|---|---|---|---|---|---|---|
| Red Type | 8 | - | 48.8 | 55.2 | - | - | - | ddeeH^{T}H^{T}pgpgCyCyDpDp |
| Pale Red | 17 | 54.8 | 20.2 | 25.0 | - | - | - | ddeeH^{T}H^{T}pg-CyCyDpDp |

| Type | | | | | | | | |
|---|---|---|---|---|---|---|---|---|
| White Type | 3 | - | - | - | - | - | - | ddeeH^{F}H^{F}pgpgCyCyDpDp |

### EXAMPLE 13

Flower petal pigment of Rhododendron ((Ericaceae) was analyzed to examine pigment genotypes of flower petal of various lines. As a result, as shown in Table 10, pigment genotypes of flower petal of various lines were clarified.. Malvidin (Mv) and petunidin (Pt), which are methylated anthocyanidins, were contained in Dpn pigment phenotype, and they were included in pigment genotype which produces Dpn. Moreover, in Cyn pigment phenotype, peonidin (Pn), which is methylated anthocyanidin, was contained, and it was included in pigment genotype which produces Cyn.

**Table 10**

| Line | Ind. | Pigment Constitution of Anthocyanidin | | | | | | Pigment Genotype |
|---|---|---|---|---|---|---|---|---|
| | | Pg(%) | Cy(%) | Pn(%) | Dp(%) | Pt(%) | Mv(%) | |
| Purple Type | 15 | - | 51.1 | 9.6 | 13.5 | 3.3 | 22.9 | ddeeH^{O}H^{O}pgpgCyCyDpDp |
| Red Type | 15 | - | 98.1 | 1.9 | - | - | - | ddeeH^{T}H^{T}pgpgCyCyDpDp |

### EXAMPLE 14

Flower petal pigment of azalea ((Ericaceae) was analyzed to examine pigment genotypes of flower petal of various lines. As a result, as shown in Table 11, pigment genotypes of flower petal of various lines were clarified. Malvidin (Mv) and petunidin (Pt), which are methylated anthocyanidins, were contained in Dpn pigment phenotype, and they were included in pigment genotype which produces Dpn. Moreover, in Cyn pigment phenotype, peonidin (Pn), which is methylated anthocyanidin, was contained, and it was included in pigment genotype which produces Cyn.

**Table 11**

| Line | Pigment Constitution of Anthocyanidin | | | | | | Pigment Genotype | CIELab color coordinate system | | |
|---|---|---|---|---|---|---|---|---|---|---|
| | Pg | Cy | Pn | Dp | Pt | Mv | | L* | C* | h |
| Rhododendron oldhamii | - | 90.3 | 9.7 | - | - | | ddeeH^{T}H^{T}pgpgCyCyDpDp | 54.9 | 60.7 | 27.5 |
| Rhododendron sp. (Hidado Akebomo) | - | 53.0 | 10.6 | 28.5 | + | 7.9 | ddeeH^{O}H^{T}pgpgCyCyDpDp | 74.8 | 31.0 | -15.7 |
| Rhododendron sp. (Hidado Miyonosakae) | - | 87.7 | 12.3 | - | - | - | ddeeH^{T}H^{T}pgpgCyCyDpDp | 74.4 | 38.2 | -13.0 |
| Rhododendron sp. (Hidado Suiaka) | | 100 | - | - | - | - | ddeeH^{T}H^{T}pgpgCyCyDpDp | 55.1 | 62.0 | 18.5 |
| Rhododendron sp. (Hidado Omurasaki) | - | 27.0 | 16.0 | 12.5 | 5.7 | 38.8 | ddeeH^{O}H^{T}pgpgCyCyDpDp | 54.7 | 59.8 | -23.2 |
| Rhododendron sp. (Hidado Sirosuna) | - | 68.6 | - | 31.4 | | | ddeeH^{O}H^{T}pgpgCyCyDpDp | 90.8 | 4.7 | 103.4 |

### EXAMPLE 15

Kinmo azalea was used as seed parent and Hirado azalea was used as pollen parent to make a crossing to create F₁ azalea. The flower petal pigment of the resultant azalea was analyzed to examine pigment genotypes and flower color inheritance of seed parent and pollen parent and their hybrids. As a result, as shown in Table 12, the pigment genotypes and flower coloration of hybridized individuals were clarified. Malvidin (Mv) and petunidin (Pt), which are methylated anthocyanidins, were contained in Dpn pigment phenotype, and they were included in pigment genotype which produces Dpn. Moreover, in Cyn pigment phenotype, peonidin (Pn), which is methylated anthocyanidin, was contained, and it was included in pigment genotype which produces Cyn.

**Table 12**

| Line | Ind.. | Pigment Constitution of Anthocyanidin | | | | | | Pigment Genotype | CIELab color coordinate system | | |
|---|---|---|---|---|---|---|---|---|---|---|---|
| | | Pg | Cy | Pn | Dp | Pt | Mv | | L* | C* | h |
| Rhododendron oldhamii (Seed Parent) x Rhododendron sp. (Hidado Akebomo) (Pollen Parent) | | | | | | | | | | | |
| KiAke97MA | 8 | - | 21.9 | 5.8 | 27.2 | 11.1 | 33.9 | ddeeH^{O}H^{T}pgpgCyCyDpDp | 54.4 | 55.0 | 2.7 |
| KiAke97mB | 6 | - | 32.3 | - | 67.7 | - | - | ddeeH^{O}H^{T}pgpgCyCyDpDp | 54.2 | 54.0 | 5.7 |
| KiAke97Ma | 12 | - | 54.9 | 45.1 | - | - | - | ddeeH^{T}H^{T}pgpgCyCyDpDp | 57.6 | 57.8 | 2.2 |
| KiAke97mB | 3 | - | 100 | - | - | - | - | ddeeH^{T}H^{T}pgpgCyCyDpDp | 58.9 | 55.8 | 17.1 |

| Rhododendron oldhamii (Seed Parent) x Rhododendron sp. (Hidado, Miyonosakae) (Pollen Parent) | | | | | | | | | | | |
|---|---|---|---|---|---|---|---|---|---|---|---|
| KiMiy97M1 | 33 | - | 75.4 | 24.6 | - | - | - | ddeeH^{T}H^{T}pgpgCyCyDpDp | 66.8 | 43.4 | 9.8 |
| KiMiy97m2 | 28 | - | 100 | - | - | - | - | ddeeH^{T}H^{T}pgpgCyCyDpDp | 81.3 | 15.5 | 12.8 |

| Rhododendron oldhamii (Seed Parent) x Rhododendron sp. (Hidado,Syuaka) (Pollen Parent) | | | | | | | | | | | |
|---|---|---|---|---|---|---|---|---|---|---|---|
| KiShu97M1 | 22 | - | 68.9 | 31.1 | - | - | - | ddeeH^{T}H^{T}pgpgCyCyDpDp | 56.1 | 58.7 | 17.3 |
| KiShu97m2 | 4 | - | 100 | - | - | - | - | ddeeH^{T}H^{T}pgpgCyCyDpDp | 58.1 | 56.6 | 18.9 |

| Rhododendron oldhamii (Seed Parent) x Rhododendron sp. (Hidado, Oomurasaki) (Pollen Parent) | | | | | | | | | | | |
|---|---|---|---|---|---|---|---|---|---|---|---|
| KiOom97MA | | - | 22.2 | 6.4 | 20.5 | 11.2 | 39.8 | ddeeH^{O}H^{T}pgpgCyCyDpDp | 55.9 | 54.8 | -3.5 |
| 6 | | | | | | | | | | | |
| KiOom97mB | 3 - | - | 29.9 | - | 70.1 | - | - | ddeeH^{O}H^{T}pgpgCyCyDpDp | 57.9 | 53.6 | -0.8 |
| KiOom97Ma | 7 | - | 48.2 | 51.8 | - | - | - | ddeeH^{T}H^{T}pgpgCyCyDpDp | 58.2 | 57.6 | 6.3 |
| KiOom97mb | 3 | - | 100 | - | - | - | - | ddeeH^{T}H^{T}pgpgCyCyDpDp | 61.3 | 53.6 | 7.6 |

| Rhododendron oldhamii (Seed Parent) x Rhododendron sp. (Hidado, Oomurasaki) (Pollen Parent) | | | | | | | | | | | |
|---|---|---|---|---|---|---|---|---|---|---|---|
| Ki8ii97MA | 5 | - | 27.2 | 10.0 | 16.1 | 10.4 | 36.3 | ddeeH^{O}H^{T}pgpgCyCyDpDp | 56.8 | 55.2 | -5.2 |
| Ki8ii97mB | 4 | - | 33.5 | - | 66.5 | - | - | ddeeH^{O}H^{T}pgpgCyCyDpDp | 57.5 | 52.8 | 3.5 |
| Ki8ii97Ma | 4 | - | 65.9 | 34.1 | - | - | - | ddeeH^{T}H^{T}pgpgCyCyDpDp | 60.9 | 53.8 | 7.6 |
| Ki8ii97mb | 1 | - | 100 | - | - | - | - | ddeeH^{T}H^{T}pgpgCyCyDpDp | 60.2 | 54.6 | 15.8 |

### EXAMPLE 16

Kurume azalea which is hose-in-hose flower, and Satsuki, which is single flower azalea were crossed to separate 144 individuals of hose-in-hose flower hybrid and 123 individuals of single flower hybrid at a ratio of 1:1. As a result, it has been clarified that genotype of Kurume azalea which has hose-in-hose flower and that of hose-in-hose flower hybrid were Dₕd (hetero) and the genotype of Satsuki, which is single flower azalea and that of single flower hybrid were dd (recessive homozygote).

### EXAMPLE 17

Flower petal pigment genotype of camellia (Camelliaceae) was examined. As a result, as shown in Table 13, flower petal pigment genotype and flower coloration of various cultivars can be understood.

**Table 13**

| Line. | Pigment Constitution of Anthocyanidin | | | Pigment Genotype | CIELab color coordinate system | | |
|---|---|---|---|---|---|---|---|
| | Pg(%) | Cy(%) | Dp(%) | | L* | C* | h |
| Toutsubaki | - | 100 | - | ddeeH^{T}H^{T}pgpgCyCyDpDp | 64.0 | 40.4 | -0.7 |
| Camellia Pitardii | - | 100 | - | ddeeH^{T}H^{T}pgpgCyCyDpDp | 58.0 | 52.3 | 3.1 |
| Sonoda Benibanayu Cha | - | 100 | - | ddeeH^{T}H^{T}pgpgCyCyDpDp | 50.3 | 58.4 | 11.8 |
| Camellia japonica, Senkaku | - | 100 | - | ddeeH^{T}H^{T}pgpgCyCyDpDp | 38.6 | 59.6 | 10.5 |
| Camellia japonica, Tamanoura | - | 100 | - | ddeeH^{T}H^{T}pgpgCyCyDpDp | 42.0 | 60.0 | 11.6 |
| Camellia japonica var. hozanensis | - | 100 | - | ddeeH^{T}H^{T}pgpgCyCyDpDp | 41.2 | 63.7 | 13.1 |

### EXAMPLE 18

Flower petal pigment genotype of Frensham which is cultivar of rose (Rosaceae) was examined. As a result, it has been proven that anthocyanidin was Cyn pigment phenotype, and D-eeH^{T}H^{T}pgpgCyCyDpDp pigment genotype.

### EXAMPLE 19

Calyx pigment genotype of "Blue Mirror", which is a cultivar of delphinium (Ranunculaceae), was examined. As a result, it has been proven that anthocyanidin was Dpn pigment phenotype, and ddeeH^{D}H^{D}pgpgCyCyDpDp pigment genotype.

### EXAMPLE 20

As for "Claret Elegance", "Saris Royalet", "Solbic Sydney", "Miss Kokura", and "Fukuoka 28 Gou", which are cultivars of carnation (Caryophyllaceae), flower petal pigment genotypes were examined. As a result, it has been proven that anthocyanidin of all of these cultivars were PgnCyn pigment phenotype and D-eeH^{T}H^{T}Pg-CyCyDpDp pigment genotype.

### Example 21

As for "Beni Kujaku", "Early Red", "Red Radiance", "Misono", and "Band Wagon", which are which are cultivars of gladiolus (Iridaceae), flower petal pigment genotypes were examined. As a result, it has been proven that anthocyanidin of all these cultivars were Pgn pigment phenotype and, ddeeH^{F}H^{F}Pg-CyCyDpDp pigment genotype.

### Example 22

As for red type cultivar chrysanthemum (Compositae), flower petal pigment genotypes were examined. As a result, it has been proven that anthocyanidin of this cultivar was Cyn pigment phenotype and ddeeH^{T}H^{T}pgpgCyCyDpDp pigment genotype.

### Example 23

Quick reference cap guides of multiple allele are shown Table 14, and Table 15, in which a row shows gametes of pollen parents and a line shows gametes of seed parents. Table 14 is a quick reference cap guide which is a combination where gene loci shown as Pg/pg, Cy/cy and Dp/dp are expressed as PgpgCyCyDpDp, and Table 15 is a quick reference cap guide which is a combination where gene loci shown as Pg/pg, Cy/cy and Dp/dp are expressed as pgpgCyCyDpDp. For example, when gene locus is PgPgCyCyDpDp, and when one multiple allele H^{O} and another multiple allele H^{O} are fertilized, and the combination becomes H^{O}H^{O}, the pigment phenotype thereof can be quickly referred from Table 14 to be PgnCynDpn.

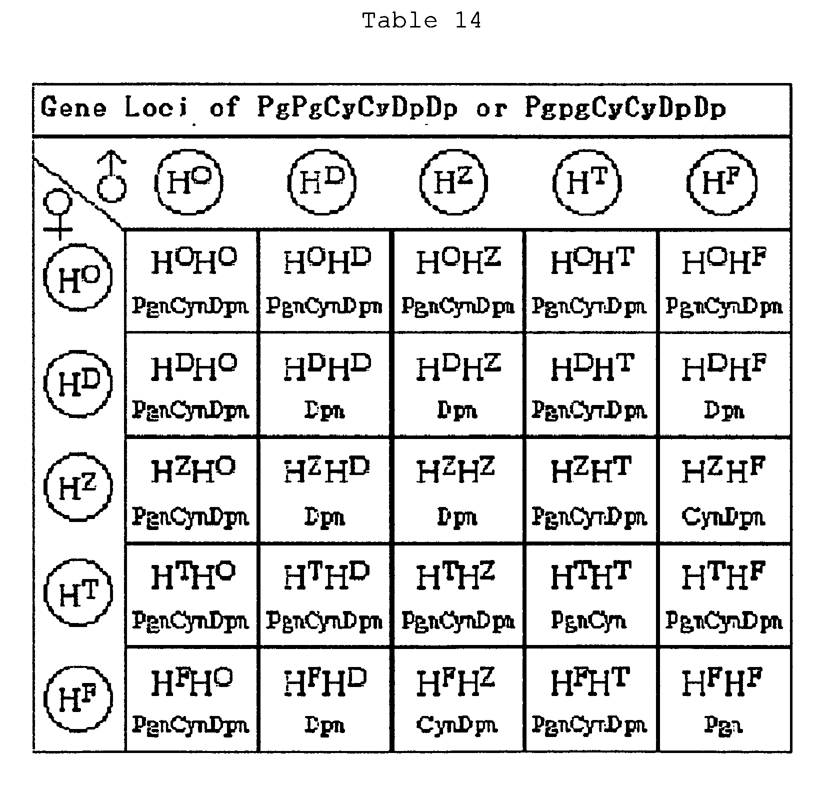

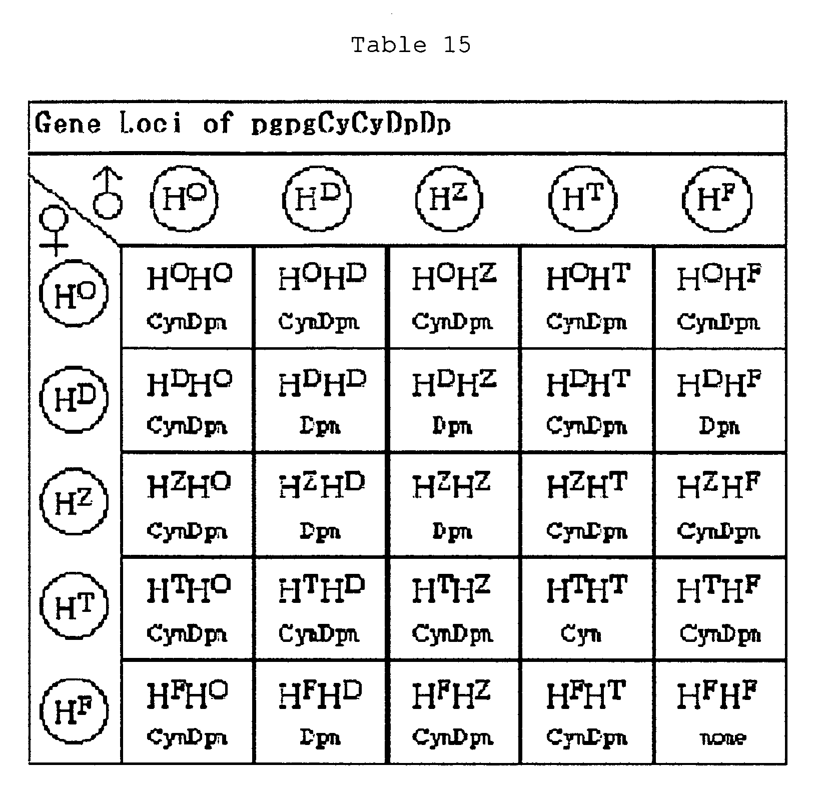

### Example 24

Table 16 shows a quick reference cap guide showing the correspondence between pigment phenotype and pigment genotype. For example, when a pigment genotype H^{T}H^{T}PgPgCyCyDpDp having PgnCyn-pigment phenotype and a pigment genotype H^{F}H^{F}pgpgCyCyDpDp of white flower (none pigment phenotype) are crossed, F₁ crossed cultivar having a pigment genotype of H^{T}H^{F}PgpgCyCyDpDp can be created, and it has be quickly referred from this quick reference cap guide that the pigment phenotype is PgnCynDpn.

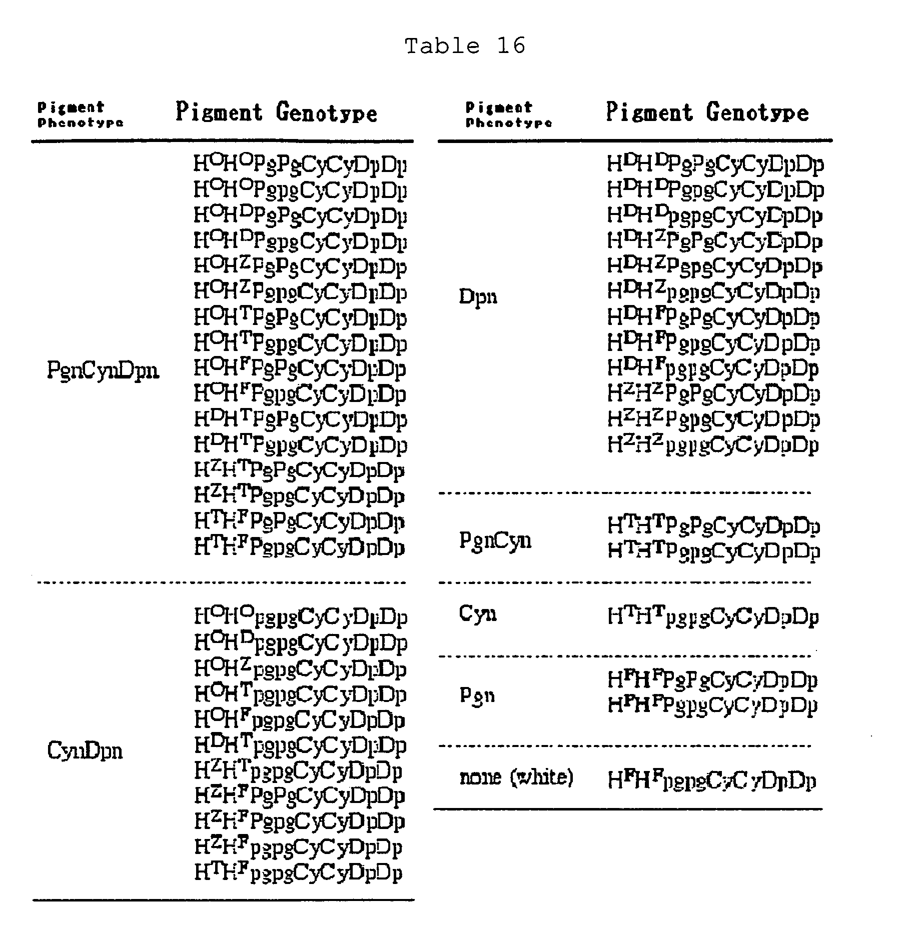

### EXAMPLE 25

Quick reference cap guides of multiple allele are shown Table 17, and Table 18, which can refer the flower coloration from multiple allele. A row shows gametes of pollen parents and a line shows gametes of seed parents.
Table 17 is a quick reference cap guide which is a combination where gene loci shown as Pg/pg,Cy/cy and Dp/dp are expressed as PgPgCyCyDpDp or PgpgCyCyDpDp, and Table 18 is a quick reference cap guide which is a combination where gene loci shown as Pg/pg, Cy/cy and Dp/dp are expressed as pgpgCyCyDpDp. For example, when gene locus is PgPgCyCyDpDp, and when one multiple allele H^{O} and another multiple allele H^{O} are fertilized, and the combination becomes H^{O}H^{O}, the pigment phenotype thereof can be quickly referred from Table 17 to be PgnCynDpn, and thus, the flower color being red purple.

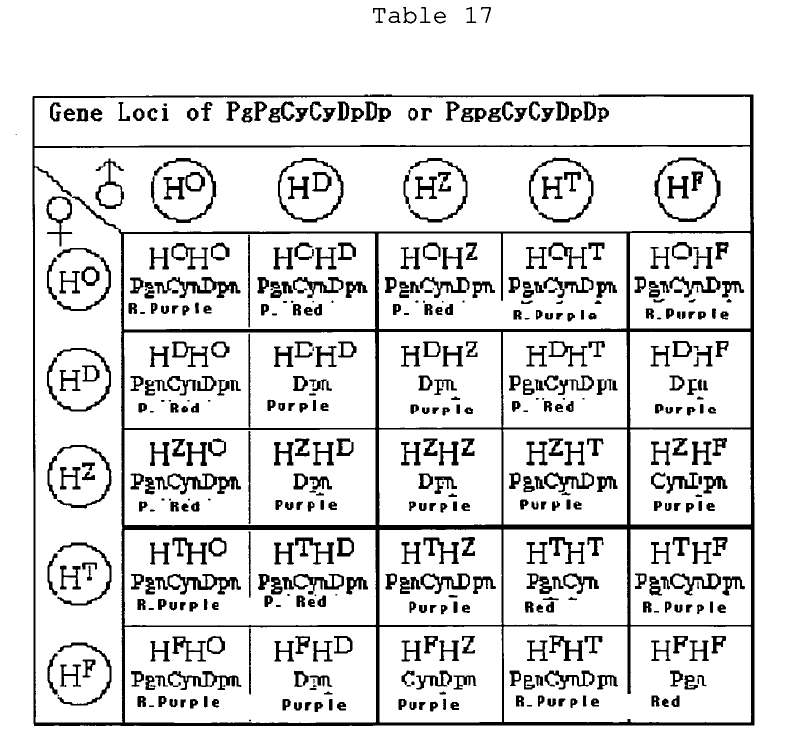

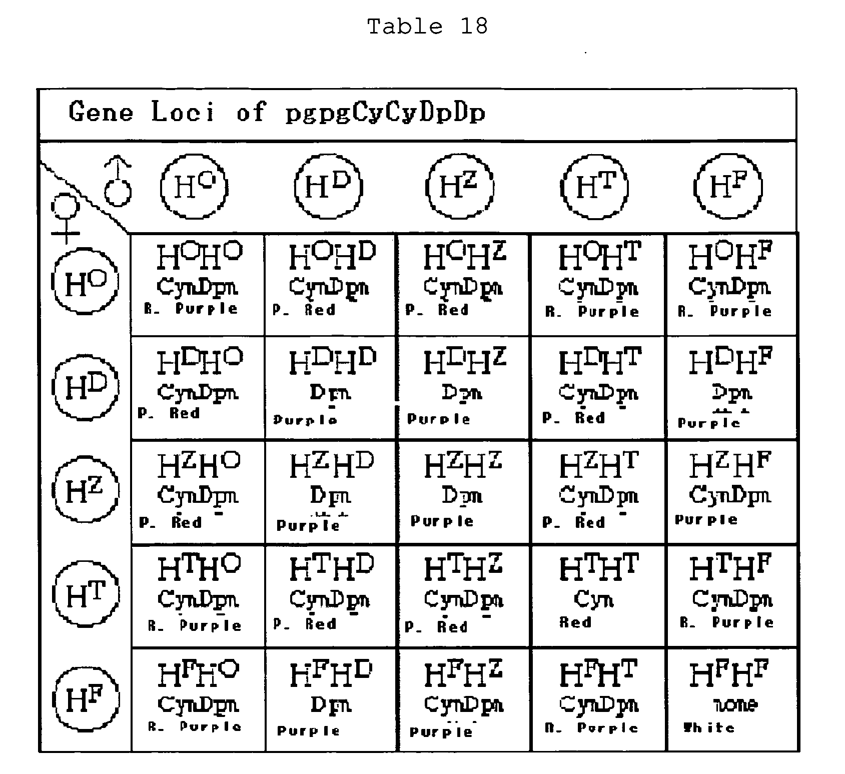

It has been clear from these examples that a breeding method in which flower color and/or flower type are reverting to pigment phenotype of genotype H^{X}H^{X}·Pg/pg·Cy/cy·Dp/dp or genotype D/d·E/e· H^{X}H^{X}·Pg/pg·Cy/cy·Dp/dp and pigment phenotype of Pgn,Cyn,Dpn is excellent a breeding method based on the flower pigment genotype.

### INDUSTRIAL APPLICABILITY

The present invention can make it possible to clarify the pigment genotype. For example, the method for crossing flowering plants based on their pigment genotypes to which genotype: D/d·E/e· H^{X}H^{X}·Pg/pg·Cy/cy·Dp/dp and pigment phenotype of Pgn, Cyn, Dpn are attributed is used, and CIELab color coordinate system of flowering plant is used to correctly measure and evaluate flower color, whereby new excellent flower color can be created.

## Claims

1. A method for crossing flowering plants based on their pigment genotypes, comprising creating new flower color utilizing new genotype H^{X}H^{X} · Pg/pg · Cy/cy · Dp/dp, which is heredity of pelargonidin (Pgn), cyanidin (Cyn), and delphinidin (Dpn), which are main flower pigments concerning the flower color expression.

2. A method for crossing flowering plants based on their pigment genotypes which creates new flower color utilizing genotype D/d·E/e·H^{X}H^{X}·Pg/pg·Cy/cy·Dp/dp , which is heredity of pelargonidin (Pgn), cyanidin (Cyn), and delphinidin (Dpn), which are main flower pigments concerning the flower color expression and which is heredity of double flower type, or marginal variegation type.

3. The method for crossing flowering plants based on their pigment genotypes according to claim 1, wherein the flower pigment genotype precipitates in and inherits flavonoid biosynthesis and has a route formula (I): 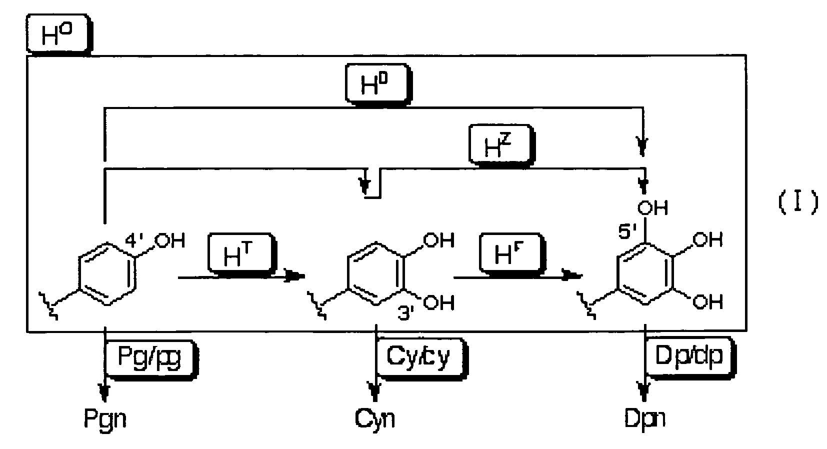 (wherein H^{T}, H^{F}, H^{D}, H^{Z}, and H^{O} are multiple alleles participating in hydroxylation of B-ring of flavonoid biosyntesis precursor participating in biosynthesis of pelargonidin (Pgn), cyanidin (Cyn), and delphinidin (Dpn). These five multiple alleles, H^{T}, H^{F}, H^{D}, H^{Z}, and H^{O}, control hydroxylation at 3'-position, hydroxylation at 5'-position, hydroxylation of 3',5'-positions, hydroxylation at 3'- and 5'-positions, and hydroxylation of 5'-, and 3',5'-position, respectively; the expression of these five multiple alleles may be other expression method, for example, T, F, D, Z, O; the expression Pg/pg,Cy/cy and Dp/dp means the existence of gene loci corresponding to the expression of dihydroflavonol reductase (DFR) or anthocyanidin synthase (AS) participating in biosynthesis of Pgn, Cyn, and Dpn; D/d is a corolla character of double flower type, and E/e is a corolla character of marginal variegation).

4. The method for crossing flowering plants based on their pigment genotypes according to claim 1, wherein flower color of the flowering plants is inherited in the course of flavonoid biosynthesis.

5. The method for crossing flowering plants based on their pigment genotypes according to claim 2, wherein flower color of the flowering plants is inherited in the course of flavonoid biosynthesis.

6. The method for crossing flowering plants based on their pigment genotypes according to claim 3, wherein flower color of the flowering plants is inherited in the course of flavonoid biosynthesis.

7. The method for crossing flowering plants based on their pigment genotypes according to any one of claims 1 to 7, wherein said flower color is maternally inherited.

8. A quick reference cap guide which determine the combination of crossing plants based on flower pigment genotype for creating a flower color, which displays the combination of multiple allele according to any one of Claims 1 to 7 taking gametes of pollen parents as a row and gametes of seed parent as a line.

9. A quick reference cap guide which determine the flower color from the combination of crossing plants based on flower pigment genotype, which displays the combination of multiple allele according to any one of Claims 1 to 7 taking gametes of pollen parents as a row and gametes of seed parent as a line to understand the flower color.

10. Use of the quick reference cap guide of multiple allele according to claim 8 for crossing based on a flower pigment genotype for creating new flower color.

11. Use of the quick reference cap guide of multiple allele according to claim 9 for crossing based on a flower pigment genotype for creating new flower color.
